Europäisches Patentamt

European Patent Office

Office européen des brevets

(19)

(11) Veröffentlichungsnummer: **0 017 059**
**B2**

(12) # NEUE EUROPÄISCHE PATENTSCHRIFT

(45) Veröffentlichungstag der neuen Patentschrift:
26.02.86

(21) Anmeldenummer: 80101368.1

(22) Anmeldetag: 17.03.80

(51) Int. Cl.⁴: **C 07 C 69/708**, C 07 C 67/26,
C 08 G 65/28, B 01 F 17/44

(54) **Verfahren zur Herstellung von alkoxylierten Fettsäuren, und deren Verwendung als Lösungsvermittler.**

(30) Priorität: 22.03.79 DE 2911241

(43) Veröffentlichungstag der Anmeldung:
15.10.80 Patentblatt 80/21

(45) Bekanntmachung des Hinweises auf die Patenterteilung:
25.11.81 Patentblatt 81/47

(45) Bekanntmachung des Hinweises auf die Entscheidung
über den Einspruch:
26.02.86 Patentblatt 86/9

(84) Benannte Vertragsstaaten:
CH DE FR GB

(56) Entgegenhaltungen:
DE - A - 1 144 232
GB - A - 924 895

Nikolaus Schönfeldt "Oberflächenaktive
Anlagerungsprodukte des Äthylenoxyds",
Wissenschaftliche Verlagsgesellschaft MBM, Stuttgart,
1959, Seite 92
Journal of the American Oil Chemists' Society, Vol. 31
(1.54)

(73) Patentinhaber: BASF Aktiengesellschaft,
Carl-Bosch-Strasse 38, D-6700 Ludwigshafen (DE)

(72) Erfinder: Oppenlaender, Knut, Dr., Otto-Dill-Strasse 23,
D-6700 Ludwigshafen (DE)
Erfinder: Krapf, Heinz, Dr., In der Schleit,
6718 Gruenstadt (DE)
Erfinder: Lang, Siegfried, Dr., Thomas-Mann-Strasse 22,
D-6700 Ludwigshafen (DE)

## Beschreibung

Zur Herstellung wäßriger Injektionslösungen von wasserunlöslichen Wirkstoffen benötigt man Lösungsvermittler (Solubilisatoren). Als solche werden Umsetzungsprodukte von Rizinusöl, hydriertem Rizinusöl oder Sorbitanestern mit Ethylenoxid (vgl. Chem. Abstr. 1957, 14213 a) verwendet, die jedoch anwendungstechnische Nachteile aufweisen.

So besitzt eine 30%ige wäßrige Lösung eines solchen Solubilisators eine Viskosität von 35 bis 40 mPa s und ist daher für viele Anwendungszwecke, wie z.B. die parenterale Applikation, zu viskos. Ein weiterer Nachteil der bekannten Solubilisatoren besteht darin, daß sie nach parenteraler Applikation zu einer Freisetzung von Histamin führen, womit ein Blutdruckabfall verbunden ist.

Weiter sind aus J. Amer. Oil Chem. Soc. 31, (1954) Ester aus 12-Hydroxystearinsäure und Polyethylenglykol bekannt, die durch Umsetzen der Säure und dem Polyol bei 160-190°C hergestellt werden und als Detergenzien dienen. Diese eignen sich jedoch nicht als Lösungsvermittler für pharmazeutische Zwecke, da sie einen zu hohen Gehalt an Dioxan besitzen.

Es wurde nun gefunden, daß in bestimmter Weise hergestellte alkoxylierte Fettsäuren diese Nachteile nicht besitzen.

Gegenstand der Erfindung ist ein Verfahren zur Herstellung von alkoxylierten Fettsäuren durch Umsetzen von Monohydroxyfettsäuren der Formel I

$CH_3$-$(CH_2)_m$ - CHOH - $(CH_2)_n$ - COOH, worin m eine ganze Zahl von 4 bis 9 und n eine ganze Zahl von 6 bis 11 darstellt, mit 15-20, vorzugsweise 14-17 Mol Ethylenoxid in Gegenwart basischer Katalysatoren, dadurch gekennzeichnet, daß man die Umsetzung bei 90-130°C durchführt.

Bevorzugt wird die Umsetzung mit Monohydroxyfettsäuren der Formel I, durchgeführt, worin m eine ganze Zahl von 5 bis 8 und n eine ganze Zahl von 7 bis 10 darstellen.

Die Umsetzung der Fettsäure mit Ethylenoxid erfolgt nach an sich bekannten Methoden in Rührautoklaven bei einem Druck von 3 bis 9 bar und einer Temperatur von 90 bis 130, vorzugsweise 100 bis 120°C (vgl. Schönfeldt, Grenzflächenaktive Ethylenoxid-Addukte, Wissenschaftl. Verlagsgesellschaft Stuttgart, 1976; DE-AS 1 901 535).

Als basische Katalysatoren eignen sich besonders Alkalihydroxide und Alkalialkoholate sowie Amine. Auch Aminoxide können verwendet werden. Beispiele für solche Katalysatoren sind: Natriumhydroxid, Kaliumhydroxid, Natriummethylat, Kaliummethylat, Natriumethylat, Kaliumethylat, Dimethyldodecylamin, Dimethyldodecylaminoxid.

Die bei der Reaktion als Katalysator wirkende Base wird nach Beendigung der Umsetzung mit einer physiologisch verträglichen Säure neutralisiert.

Die Erfindung betrifft insbesondere die Verwendung der er-findungsgemäß erhaltenen Produkte als Lösungsvermittler, vorzugsweise für pharmazeutische Zwecke.

Die Verwendung der erfindungsgemäß erhaltenen Verbindungen als Lösungsvermittler erfolgt in bekannter Weise. So wird beispielsweise der Wirkstoff mit diesen gemischt und - gegebenenfalls unter leichtem Erwärmen- Wasser zugesetzt. Geeignete Wirkstoffe sind z.B. fettlösliche Vitamine (Vitamin A, E, K), Steroide und Benzodiazepine.

Die Viskosität einer 30%igen wäßrigen Lösung der erfindungs-gemäß erhaltenen Verbindungen liegt bei 3,5 bis 15 mPa s und ist damit erheblich niedriger als entsprechende Lösungen der bekannten Lösungsvermittler Polyoxyethylen(20)-sorbitanmonooleat, Glycerinpolyglykolricinoleat oder Glycerinpolyglykoloxistearat. Darüber hinaus führen die erfindungsgemäß erhaltenen Substanzen im Gegensatz zu den bekannten Lösungsvermittlern nach parenteraler Gabe an Säugetiere zu keiner Histaminfreisetzung und damit auch nicht zu einem Blutdruckabfall.

## Beispiel 1

156 Gewichtsteile (0,52 Molteile) eines Gemisches aus 9- und 10-Hydroxystearinsäure werden in Gegenwart von 1,5 Gewichtsteilen KOH-Pulver in üblicher WEise im Autoklaven mit 343 Gewichtsteilen (7,6 Molteilen) Ethylenoxid bei 110 bis 120°C umgesetzt. Man erhält eine gelbliche viskose Flüssigkeit. Das als Katalysator verwendete KOH-Pulver wird mit Phosphorsäure neutralisiert. Das Reaktionsprodukt besitzt die Verseifungszahl 60, die Hydroxylzahl 123 und eine Viskosität von 8,2 (30%ige Lösung). Eine 1%ige Lösung der so erhaltenen Substanz in 5%iger Kochsalzlösung besitzt den Trübungspunkt 51 bis 52°C.

## Beispiel 2

165 Gewichtsteile (0,55 Molteile) 12-Hydroxystearinsäure werden in Gegenwart von 0,5 Gewichtsteilen einer 50%igen KOH-Lösung mit 339 Gewichtsteilen (7,7 Molteilen) Ethylenoxid bei 100 bis 110°C im Autoklaven umgesetzt. Man erhält eine nahezu farblose weiche Paste. Das Kaliumhydroxid wird mit Essigsäure neutralisiert. Das Reaktionsprodukt besitzt einen Auftaupunkt von 25 bis 26°C, die Verseifungszahl 61, die Hydroxylzahl 125 und eine Viskosität von 11,5 mPa s (30%ige Lösung). Der Trübungspunkt einer 1%igen Lösung des Produkts in 5%iger Kochsalzlösung liegt bei 54 bis 55°C.

### Beispiel 3

1050 Gewichtsteile (3,5 Molteile) 12-Hydroxystearinsäure werden unter Zusatz von 31,5 Gewichtsteilen N,N-Dimethyldodecylamin als Katalysator mit 154 Gewichtsteilen (3,5 Molteilen) Ethylenoxid bei 100 bis 110°C im Autoklaven umgesetzt. 176,5 Gewichtsteile der so erhaltenen gelblichen festen Substanz, welche 1 Mol Ethylenoxid pro Mol 12-Hydroxy-stearinsäure enthält, werden in Gegenwart von 1,8 Gewichtsteilen KOH-Pulver im Autoklaven bei 100 bis 110°C mit 330 Gewichtsteilen (7,5 Molteilen) Ethylenoxid umgesetzt. Man erhält eine schwach gelbliche weiche Paste, die mit Essigsäure auf pH 7 eingestellt wird. Das Reaktionsprodukt besitzt die Verseifungszahl 54, die Hydroxylzahl 123 und eine Viskosität von 6,5 (30%uge Lösung).

Der Trübungspunkt einer 1%igen Lösung des Produkts in 5%iger Kochsalzlösung liegt bei 57 bis 58°C.

### Anwendungsbeispiele

a) 500 mg Propanidid werden bei 50 bis 60°C mit 2 g des gemäß Beispiel 1 erhaltenen Ethoxylierungsproduktes gemischt. Unter Rühren werden bei derselben Temperatur 7,5 ml Wasser zugegeben. Man erhält eine klare wäß-rige Lösung, die eine Viskosität von 9 mPa s (Pa s = Pascal-Sekunden) aufweist.

b) 1 g Vitamin-E-Acetat wird mit 2 g des gemäß Beispiel 2 erhaltenen Lösungsvermittlers gemischt, auf 50 bis 60°C erwärmt und bei dieser Temperatur langsam mit 7 ml Wasser vermischt. Die so erhaltene klare Lösung besitzt eine Viskosität von 4,5 mPa s.

c) 1,5 Azulen werden mit 2,0 g Lösungsvermittler gemäß Beispiel 3 gemischt und unter leichtem Erwärmen mit 6,5 ml Wasser vermischt. Man erhält eine klare, tiefblaue wäßrige Lösung mit der Viskosität 4,8 mPa s.

d) 800 mg Vitamin $K_1$ werden mit 2 g Lösungsvermittler gemäß Beispiel 3 gemischt. Unter Erwärmen auf 60°C werden langsam 7,5 ml 60°C warmes Wasser zugegeben. Die klare Vitamin-$K_1$-Lösung weist eine Viskosität von 4 mPa s auf.

### Patentansprüche

1. Verfahren zur Herstellung von alkoxylierten Fettsäuren durch Umsetzen von Monohydroxyfettsäuren der Formel I
$$CH_3\text{-}(CH_2)_n\text{ - }CHOH\text{ - }(CH_2)_n\text{ - }COOH,$$
worin m eine ganze Zahl von 4 bis 9 und n eine ganze Zahl von 6 bis 11 darstellt, mit 12-20, vorzugsweise 14-17 Mol Ethylenoxid in Gegenwart basischer Katalysatoren, <u>dadurch gekennzeichnet</u>, daß man die Umsetzung bei 90-130°C durchführt.

2. Verwendung der gemäß Anspruch 1 erhältlichen alkoxylierten Fettsäuren als Lösungsvermittler.

### Claims

1. A process for the preparation of alkoxylated fatty acids by reacting monobydroxy-fatty acids of the formula 1
$$CH_3\text{-}(CH_2)_m\text{-}CHOH\text{-}(CH_2)_n\text{-}COOH,$$
where m is an integer from 4 to 9 and n is an integer from 6 to 11, with from 12 to 20, preferably from 14 to 17, moles of ethylene oxide in the presence of a basic catalyst, wherein the reaction is carried out at from 90 to 130°C.

2. The use of the alkoxylated fatty acids obtainable according to claim 1 as solubilizers.

### Revendications

1. Procédé de preparation d'acides gras alcopyles par réaction de monohydroxyacides gras de formule **1**
$$CB_3\text{-}(CH_2)_m\text{-}CHOH\text{-}(CB_2)_n\text{-}COOH$$
où <u>m</u> représente un nombre entier de 4 à 9 et <u>n</u> un nombre entier de 6 à 11, avec 12-20, moles, de preference 14 à 17 moles, d'éthylèneoxyde en présence de catalyseurs basiques, caractérisé par le fait qu'on effectue la réaction à 90 - 130°C.

2. Utilisation des acides gras alcoxylés, obtenus selon la revendication 1, comme tiers-solvants.